# FASCICULE DE BREVET EUROPEEN

(11) **EP 2 874 990 B1**
(45) Date de publication et mention de la délivrance du brevet: **31.05.2017**
(21) Numéro de dépôt: 13735294.4
(22) Date de dépôt: 11.07.2013
(51) Int. Cl.: C07C 51/56, C07C 51/573

(54) **SYNTHÈSE D'ANHYDRIDE (MÉTH)ACRYLIQUE PAR TRANSANHYDRIFICATION**
SYNTHESE VON (METH)ACRYLSÄUREANHYDRID DURCH TRANSANHYDRISIERUNG
SYNTHESIS OF (METH)ACRYLIC ANHYDRIDE BY TRANSANHYDRIZATION

(30) Priorité: 18.07.2012 FR 1256940
(43) Date de publication de la demande: 27.05.2015
(73) Titulaire: Rhodia Operations, 75009 Paris (FR)
(72) Inventeur: ARDAUD, Pierre, Marcel, F-69110 Ste Foy Les Lyon (FR); RACHED, Rabih, Julien, F-69390 Millery (FR); VIDAL, Thierry, Alban, Marcel, F-69003 Lyon (FR)
(74) Mandataire: Cordier, Pascal Christian
(86) Numéro de dépôt international: PCT/EP2013/064698
(87) Numéro de publication internationale: WO 2014/012843

(56) Documents cités:
- EP-A1- 0 196 520
- WO-A1-2009/098422
- GB-A- 538 310
- US-A1- 2002 161 260
- US-A1- 2009 264 673

## Description

La présente invention concerne un procédé de préparation d'un anhydride (méth)acrylique par réaction d'un acide (méth)acrylique avec un anhydride autre qu'un anhydride (méth)acrylique, selon une réaction dite de transanhydrification.

Les anhydrides (méth)acryliques sont classiquement préparés par une réaction de transanhydrification, typiquement par réaction d'acide (méth)acrylique avec de l'anhydride acétique, ce par quoi il se forme de l'acide acétique et l'anhydride recherché. L'acide acétique formé est généralement éliminé par distillation au fur et à mesure de sa formation. Ce type de réaction, bien connu, est décrit par exemple dans la demande EP 1 273 565.

En marge de la préparation de l'anhydride (méth)acrylique souhaité, il se forme des sous-produits, notamment des produits de polymérisation et des produits d'addition, qui nécessitent une purification de l'anhydride (méth)acrylique formé. La teneur en ces sous-produits peut être réduite de façon connue en soi par l'addition d'inhibiteurs de polymérisation. Néanmoins, même en employant de tels inhibiteurs, il continue à se former des sous-produits. Typiquement, ces sous-produits sont éliminés par distillation, ce qui est une opération délicate, notamment compte tenu du caractère lacrymogène de l'anhydride (méth)acrylique.

Des procédés de synthèse d'anhydride (méth)acrylique de type « batch » (procédés discontinus par lots) ont notamment été décrits, par exemple dans la demande EP 0 231 689.

Alternativement, des procédés continus et semi-continus, plus intéressants, ont également été proposés, notamment dans EP 1 237 565 ou US 2009/0264673, et permettent de réduire la présence de sous-produits.

Par ailleurs, il a été envisagé l'emploi de catalyseurs pour améliorer la réaction. Dans ce cadre, pour l'essentiel, ce sont des catalyseurs hétérogènes qui ont été envisagés, notamment dans US 2002/0161260, et qui peuvent poser des difficultés en termes d'extrapolation ou de transfert de matière. Plus ponctuellement, il a été proposé des catalyseurs homogènes, comme par exemple l'acide sulfurique ou les acides sulfoniques décrit dans DE 3510035, EP 0196520, GB 538310, ou WO 2009/098422, qui ne posent pas ce type de difficulté, mais qui, en contrepartie, présentent le plus souvent un inconvénient majeur, à savoir qu'ils impliquent en général des étapes lourdes de post-traitement pour être séparés de l'anhydride. Ainsi, ces catalyseurs ne conduisent pas systématiquement à une amélioration du rendement et présentent en outre souvent l'inconvénient de devoir être éliminés à l'issue de la réaction. Un but de la présente invention est de fournir un procédé de préparation d'anhydride (méth)acrylique évitant des étapes lourdes de post-traitement de l'anhydride, notamment des étapes lourdes d'élimination du catalyseur utilisé.

La présente invention vise aussi à fournir un procédé efficace de préparation d'anhydride (méth)acrylique, qui puisse, au besoin, être mis en oeuvre selon un mode continu, en évitant les lourdeurs et inconvénients des procédés de type « batch ».

A cet effet, la présente invention propose de mettre en oeuvre un catalyseur particulier, à savoir un catalyseur acide plus volatil que l'anhydride (méth)acrylique synthétisé.

Plus précisément, la présente invention a pour objet un procédé de préparation d'un anhydride de formule A-C(=O)-O-(O=)C-A, où A est -CR=CH₂ et R est -H ou -CH₃, comprenant :
a) une étape de réaction d'un anhydride B-C(=O)-O-(O=)C-B avec un acide A-COOH, A étant tel que défini ci-dessus, cette étape conduisant à la formation d'un anhydride A-C(=O)-O-(O=)C-B et d'un acide B-COOH, A et B étant tels que ledit acide B-COOH est plus volatil que ledit acide A-COOH, et
b) une étape de réaction dudit anhydride A-C(=O)-O-(O=)C-B avec l'acide A-COOH dans des conditions telles que la quantité d'acide B-COOH est inférieure à la quantité d'acide A-COOH, conduisant à la formation de l'anhydride A-C(=O)-O-(O=)C-A,
dans lequel lesdites étapes de réaction sont mises en oeuvre en présence d'un catalyseur acide plus volatil que ledit anhydride A-C(=O)-O-(O=)-C-A, et choisi parmi l'acide trifluoroacétique, le triflimide et l'acide triflique. Dans le cadre de la présente description, les anhydrides répondant à la formule A-C(=O)-O-(O=)C-A, où A est -CR=CH₂ et R est -H ou -CH₃, sont désignés par le terme générique d'anhydrides « (méth)acryliques ». Un anhydride (méth)acrylique au sens de l'invention désigne également un mélange de tels anhydrides.

Plus généralement, le terme « (méth)acrylique » est employé dans la présente description comme synonyme de l'expression « acrylique et/ou méthacrylique ». Ainsi, lorsqu'il est fait référence à un acide (méth)acrylique, ce terme désigne l'acide acrylique CH₂=CH-COOH ou l'acide méthacrylique CH₂=C(CH₃)-COOH, ou bien encore un mélange de ces deux acides. De la même façon, si on fait référence à un anhydride (méth)acrylique, on entend désigner un anhydride acrylique CH₂=CH-C(=O)-O-(O=)C-CH=CH₂, un anhydride méthacrylique CH₂=C(CH₃)-C(=O)-O-(O=)C-C(CH₃)=CH₂, un anhydride mixte acrylique et méthacrylique CH₂=CH-C(=O)-O-(O=)C-C(CH₃)=CH₂, ou bien un mélange de ces anhydrides.

Le procédé de l'invention permet de préparer les différentes espèces répondant au terme « anhydride (méth)acrylique », à savoir, au choix, de l'anhydride acrylique, de l'anhydride méthacrylique ou un mélange des deux, par choix de l'acide A-COOH employé dans le procédé.

Selon un mode de réalisation intéressant, l'anhydride (méth)acrylique préparé selon l'invention est soit un anhydride acrylique CH₂=CH-C(=O)-O-(O=)C-CH=CH₂, soit un anhydride méthacrylique CH₂=C(CH₃)-C(=O)-O-(O=)C-C(CH₃)=CH₂, respectivement en partant d'acide acrylique ou d'acide méthacrylique à titre d'acide A-COOH. Il n'est toutefois pas exclu, selon un mode de réalisation plus particulier, de partir d'un mélange d'acides acrylique et méthacrylique.

Selon l'invention, le procédé de préparation de l'anhydride (méth)acrylique comprend une étape, notée a), de réaction d'un anhydride B-C(=O)-O-(O=)C-B avec un acide A-COOH, où A et B sont tels que l'acide B-COOH est plus volatil que l'acide A-COOH.

Au sens de la présente invention, l'expression « un premier composé est plus volatil qu'un second composé » signifie que le premier composé a une température d'ébullition plus basse que celle du second composé, dans les conditions de pression de l'étape considérée.

Le choix d'un anhydride B-C(=O)-O-(O=)C-B tel que B-COOH est plus volatil que A-COOH, conduit à ce que chacun des produits qui peuvent se former au cours de l'étape de réaction soit plus volatil que l'anhydride (méth)acrylique A-C(=O)-O-(O=)C-A. Ce dernier, moins volatil, est alors généralement plus facile à isoler du reste des composés présents, lors d'étapes de traitement ultérieures.

Cette étape a) conduit à la formation d'un composé de type anhydride mixte A-C(=O)-O-(O=)C-B, plus volatil que l'anhydride A-C(=O)-O-(O=)C-A, et d'un acide B-COOH.

Selon l'invention, le procédé de préparation de l'anhydride (méth)acrylique comprend également une étape, notée b), de réaction dudit anhydride A-C(=O)-O-(O=)C-B avec l'acide A-COOH dans des conditions telles que la quantité d'acide B-COOH est inférieure à la quantité d'acide A-COOH.

Dans de telles conditions, l'étape b) conduit à la formation de l'anhydride A-C(=O)-O-(O=)C-A.

En effet, lorsque la quantité d'acide B-COOH est inférieure à la quantité d'acide A-COOH, l'équilibre de la réaction tend vers la formation de l'anhydride souhaité. Ainsi, l'anhydride mixte A-C(=O)-O-(O=)C-B réagit avec l'acide A-COOH, conduisant à la formation de l'anhydride A-C(=O)-O-(O=)C-A et d'acide B-COOH.

Si de telles conditions ne sont pas vérifiées, alors l'anhydride mixte ne peut pas réagir avec l'acide A-COOH pour former l'anhydride A-C(=O)-O-(O=)C-A, il peut donc se retrouver parmi les produits finaux.

Ainsi, au cours du procédé, lorsque la quantité d'acide B-COOH devient supérieure à la quantité d'acide A-COOH, la deuxième étape de réaction ne peut plus avoir lieu. On obtient alors parmi les produits finaux à la fois de l'anhydride A-C(=O)-O-(O=)C-A et de l'anhydride mixte A-C(=O)-O-(O=)C-B. Le fait que ce dernier soit plus volatil que l'anhydride A-C(=O)-O-(O=)C-A permet de le séparer aisément de celui-ci.

Dans le cadre de l'invention, les étapes de réaction a) et b) peuvent se dérouler simultanément ou de façon séquentielle.

L'ensemble des deux étapes de réaction a) et b) du procédé selon l'invention peut être désigné par « réaction de transanhydrification ».

Ainsi, l'anhydride (méth)acrylique est formé selon une réaction de transanhydrification, c'est-à-dire partant de l'acide (méth)acrylique et d'un anhydride B-C(=O)-O-(O=)C-B, différent de l'anhydride souhaité, et conduisant à l'anhydride (méth)acrylique souhaité et un acide carboxylique B-COOH, différent de l'acide initial, avec comme composé intermédiaire entre les anhydrides initial et final, l'anhydride A-C(=O)-O-(O=)C-B.

On peut associer à cette réaction de transanhydrification une zone de réaction qui délimite l'espace dans lequel se déroulent les deux étapes a) et b).

Ainsi, différents composés peuvent être présents dans la zone de réaction et en sortie de cette zone.

Plus précisément, à un instant donné, la zone de réaction comprend généralement de l'anhydride B-C(=O)-O-(O=)C-B, de l'acide A-COOH, le catalyseur, le composé mixte A-C(=O)-O-(O=)C-B, de l'acide B-COOH et de l'anhydride A-C(=O)-O-(O=)C-A. L'ensemble de ces composés peut également être présent en sortie de la zone de réaction.

En effet, comme indiqué plus haut, les réactifs B-C(=O)-O-(O=)C-B et A-COOH, ainsi que le catalyseur, réagissent dans la zone de réaction pour d'abord former l'anhydride mixte A-C(=O)-O-(O=)C-B et l'acide B-COOH. Puis, si la quantité d'acide B-COOH est inférieure à celle d'acide A-COOH, l'anhydride mixte réagit alors avec l'acide A-COOH et de l'anhydride A-C(=O)-O-(O=)C-A est formé.

Selon l'invention, les étapes a) et b) sont mises en oeuvre en présence d'un catalyseur acide plus volatil que l'anhydride A-C(=O)-O-(O=)C-A synthétisé.

Le choix d'un catalyseur acide plus volatil permet une élimination extrêmement aisée du catalyseur d'une part et de l'anhydride synthétisé d'autre part, par simple évaporation du catalyseur, ce qui est particulièrement avantageux. L'anhydride synthétisé est alors obtenu sous une forme purifiée et peut éventuellement être davantage purifié, dans le cas où une certaine quantité de catalyseur est encore présente, par un traitement additionnel, au charbon actif par exemple. Le catalyseur acide est choisi parmi l'acide trifluoroacétique (TFA) de formule CF₃-COOH, le trifluorométhane sulfonimide, également appelé triflimide, de formule (CF₃-SO₂)₂-NH, et l'acide trifluorométhane sulfonique, également appelé acide triflique, de formule CF₃-SO₂-OH.

De préférence, le catalyseur acide utilisé est l'acide triflique.

L'acide triflique a pour avantage d'être un catalyseur particulièrement réactif, induisant un faible temps de séjour dans la zone de réaction et autorisant la mise en oeuvre du procédé en continu, comme expliqué plus loin dans la description.

Selon l'invention, l'acide B-COOH formé est plus volatil que l'anhydride A-C(=O)-O-(O=)C-A formé. La séparation de l'anhydride à l'issue de la réaction est alors très aisée, étant donné qu'à la fois le catalyseur et l'acide formé sont plus volatils que l'anhydride formé.

Selon l'invention, l'anhydride B-C(=O)-O-(O=)C-B et l'acide A-COOH sont plus volatils que l'anhydride préparé. L'anhydride formé est ainsi moins volatil que les réactifs, ce qui facilite son isolement, même lorsqu'une certaine quantité de réactifs n'a pas réagi et se retrouve parmi les composés formés pendant la réaction.

Ainsi, l'anhydride B-C(=O)-O-(O=)C-B, l'acide A-COOH et l'acide B-COOH sont plus volatils que l'anhydride A-C(=O)-O-(O=)C-A formé. L'anhydride formé est donc le composé le moins volatil parmi les produits et réactifs et son isolement est d'autant plus facilité.

Selon un mode de réalisation, l'acide B-COOH est éliminé au cours des étapes a) et b).

En effet, comme mentionné plus haut, au cours de l'étape b) l'anhydride mixte A-C(=O)-O-(O=)C-B peut réagir avec l'acide A-COOH pour donner l'anhydride souhaité A-C(=O)-O-(O=)C-A, si la quantité d'acide B-COOH est inférieure à celle d'acide A-COOH. Par conséquent, afin d'obtenir de telles conditions et de déplacer l'équilibre de la réaction vers la formation de l'anhydride souhaité, l'acide B-COOH formé peut être éliminé au cours des étapes a) et b), c'est-à-dire retiré de la zone de réaction.

L'élimination progressive de l'acide B-COOH permet ainsi d'obtenir des conditions permettant la formation de l'anhydride souhaité A-(C=O)-O-(C=O)-A.

L'élimination de l'acide B-COOH est par exemple effectuée par distillation.

Selon un mode de réalisation, le catalyseur acide est moins volatil que l'acide B-COOH.

Ce mode est avantageux car le catalyseur acide est alors non seulement séparable de l'anhydride A-C(=O)-O-(O=)C-A synthétisé, mais également de l'acide B-COOH formé. Le catalyseur peut ainsi être directement recyclable et réutilisable dans les étapes de réaction, comme décrit plus loin.

Lorsque le catalyseur acide utilisé est plus volatil que l'acide B-COOH, le catalyseur peut également être recyclé par une étape de traitement ultérieur des effluents contenant l'acide B-COOH.

Le catalyseur peut donc être récupéré, recyclé et réutilisé dans le procédé selon l'invention.

Selon un mode de réalisation, les étapes a) et b) du procédé sont effectuées en continu.

Dans le cadre de la présente description, on entend par « procédé effectué en continu » ou plus simplement « procédé continu » un procédé dont les différentes opérations successives s'enchaînent sans interruption et par conséquent dans lequel le produit, ici l'anhydride (méth)acrylique, est élaboré de façon ininterrompue.

Ainsi, selon ce mode, les réactifs sont introduits de façon continue et les composés susceptibles d'être obtenus sont récupérés de façon continue également. Le procédé de l'invention peut donc soit être mis en oeuvre de façon non continue, on le désigne alors par « procédé batch » ou « procédé en mode batch » (par lots), soit de façon continue, on la désigne alors par « procédé continu ».

Selon un mode de réalisation, la masse molaire de B est inférieure à la masse molaire de A.

Avantageusement, B est un groupe méthyle ou éthyle.

De préférence, B est un groupe méthyle.

La préparation de l'acide (méth)acrylique est ainsi typiquement effectuée par réaction d'un acide (méth)acrylique avec l'anhydride acétique.

Par exemple, l'acide méthacrylique est préparé par réaction de l'acide méthacrylique avec l'anhydride acétique.

Selon un mode de réalisation, les étapes de réaction a) et b) s'effectuent à une température de 60°C à 120°C, avantageusement de 70°C à 110°C, de préférence de 90°C à 100°C.

En effet, plus la température augmente, plus la cinétique de réaction est rapide. En revanche, plus la température augmente, plus les composés présents se dégradent.

Ainsi, la température des étapes de réaction est choisie afin d'avoir une cinétique suffisamment rapide, tout en évitant une dégradation trop importante des composés.

Selon un mode de réalisation, les étapes de réaction a) et b) s'effectuent à une pression de 0,01 bar à 3 bar, avantageusement de 0,5 bar à 1,5 bar, de préférence à pression atmosphérique.

Au sens de la présente invention, on entend par « pression atmosphérique » la pression ambiante qui règne dans les conditions du procédé, égale à 1 bar ou avoisinant 1 bar.

Le procédé peut être mis en oeuvre quelle que soit la pression, mais il est particulièrement avantageux de travailler à pression atmosphérique, étant donné que cela permet de s'affranchir de tout contrôle de la pression.

Avantageusement, le ratio molaire entre l'acide A-COOH et l'anhydride B-C(=O)-O-(O=)C-B est de 0,5 à 5, avantageusement de 1,5 à 3, de préférence inférieur à 2,5 et encore plus préférentiellement inférieur à 2.

Avantageusement, le ratio entre la masse de catalyseur acide et la masse totale des réactifs B-C(=O)-O-(O=)C-B et A-COOH est de 5 ppm à 1%, avantageusement de 20 ppm à 100 ppm.

Selon un mode de réalisation, le procédé comprend une étape d'extraction du catalyseur acide et de l'acide B-COOH formé, pour séparer le catalyseur acide et l'acide B-COOH, notamment par distillation.

Cette étape d'extraction se déroule après les étapes a) et b).

A l'issue de la réaction de transanhydrification, les principaux composés présents sont le catalyseur acide, l'acide B-COOH et l'anhydride A-C(=O)-O-(O=)C-A. Ainsi, le catalyseur acide et l'acide B-COOH formé sont extraits notamment par distillation afin d'isoler l'anhydride A-C(=O)-O-(O=)C-A et de recycler le cas échéant le catalyseur pour qu'il soit réutilisable. Dans ce cas, le catalyseur peut être réutilisé dans l'étape de réaction susmentionnée.

On peut ainsi définir une zone d'extraction délimitant l'espace dans lequel se déroule l'étape d'extraction. Les zones de réaction et d'extraction peuvent être distinctes, confondues ou se chevaucher, comme décrit plus loin.

Avantageusement, après l'étape d'extraction, le catalyseur acide est récupéré et recyclé pour être utilisé dans l'étape de réaction de l'acide A-COOH avec l'anhydride B-C(=O)-O-(O=)C-B.

Divers traitements permettant de récupérer le catalyseur dans le mélange catalyseur/acide B-COOH extrait après l'étape de réaction sont envisageables. De préférence, la récupération du catalyseur est effectuée par distillation.

Comme indiqué plus haut, lorsque le catalyseur acide est moins volatil que l'acide B-COOH, la séparation entre le catalyseur et l'acide B-COOH est plus facile. Cette séparation est typiquement effectuée par distillation.

Les étapes de réaction sont généralement effectuées dans un réacteur.

Ce réacteur définit alors la zone de réaction. Il s'agit généralement d'un réacteur piston ou d'un réacteur continu agité, ou encore d'une cascade de réacteurs continus agités.

Avantageusement, l'étape d'extraction est effectuée dans une colonne de distillation.

Cette colonne définit alors la zone d'extraction.

La colonne possède par exemple de 10 à 30 plateaux théoriques, et plus précisément par exemple 20 plateaux théoriques.

Du fait des différences de volatilité entre les différents composés obtenus, l'anhydride (méth)acrylique, le composé le moins volatil de tous, est récupéré en pied de colonne, tandis que le catalyseur acide et l'acide B-COOH sont récupérés en tête de colonne. Si, de plus, le catalyseur est moins volatil que l'acide B-COOH, il est récupéré avant l'acide B-COOH qui, lui, est récupéré en tête de colonne.

L'étape d'extraction peut également être effectuée dans plusieurs colonnes successives, reliées entre elles.

Selon un mode de réalisation, les étapes de réaction a) et b) sont effectuées dans un réacteur et l'étape d'extraction est effectuée dans une ou plusieurs colonnes de distillation successives, distinctes du réacteur.

Ainsi, les divers réactifs, à savoir B-C(=O)-O-(O=)C-B, A-COOH et le catalyseur, réagissent au sein du réacteur, dans la zone de réaction, pour former l'anhydride A-C(=O)-O-(O=)C-B, l'acide B-COOH, et l'anhydride (méth)acrylique souhaité de façon quantitative si les conditions requises sont vérifiées. Le réacteur étant relié à la colonne de distillation, au fur et à mesure que se déroulent les étapes a) et b) dans le réacteur, les différents produits (A-C(=O)-O-(O=)C-A, B-COOH et éventuellement A-C(=O)-O-(O=)C-B) et/ou les réactifs migrent vers la colonne de distillation, définissant la zone d'extraction, qui permet de séparer l'anhydride formé des autres composés présents.

Le réacteur et la colonne sont alors des entités distinctes.

Ainsi, même si le réacteur est distinct de la colonne, lorsque les deux entités sont reliées, la réaction de transanhydrification peut également se dérouler dans la colonne. Les zones de réaction et d'extraction sont donc alors initialement distinctes, mais peuvent se chevaucher.

L'avantage de ce mode réside dans la possibilité de choisir les paramètres de réaction au sein du réacteur (tels que la température, la pression ou les quantités de réactifs) indépendamment des conditions de séparation dans la colonne.

Selon un autre mode de réalisation, les étapes de réaction a) et b) et l'étape d'extraction sont effectuées dans une ou plusieurs colonnes de distillation successives. Selon ce mode, les trois étapes sont effectuées au sein d'une seule et même enceinte. Les zones de réaction et d'extraction sont donc alors confondues.

Ainsi, les divers réactifs sont directement introduits au sein de la colonne, par exemple au milieu de la colonne, et réagissent afin de former l'anhydride (méth)acrylique, ainsi que les autres produits (B-COOH, A-C(=O)-O-(O=)C-B). Au fur et à mesure que ces composés se forment, ils sont soumis à l'étape d'extraction qui permet de les séparer. On parle alors de distillation réactive.

Selon ce mode, même si le catalyseur est plus volatil que l'anhydride A-C(=O)-O-(O=)C-A, il est généralement entraîné vers le pied de colonne, mélangé à l'anhydride A-C(=O)-O-(O=)C-A. La séparation de ces deux composés peut alors être effectuée de façons variées, notamment aisément par distillation, étant donné que le catalyseur est plus volatil que l'anhydride (méth)acrylique.

Comme indiqué plus haut, selon un mode de réalisation, le catalyseur est l'acide triflique. Or, l'acide triflique est particulièrement adapté au procédé selon l'invention, que celui-ci soit en mode « batch » (par lots) ou en mode continu.

En effet, l'acide triflique est particulièrement réactif et conduit à une formation rapide de l'anhydride (méth)acrylique. Ainsi, à la sortie du réacteur ou directement au sein de la colonne de distillation, on obtient rapidement une quantité significative d'anhydride mixte A-C(=O)-O-(O=)C-B. Cela permet donc, après extraction du catalyseur et de l'acide B-COOH, d'obtenir un rendement significatif d'anhydride (méth)acrylique, typiquement supérieur à 50%, avantageusement supérieur à 75%, de préférence de 80% à 95% par rapport à la quantité d'anhydride B-C(=O)-O-(O=)C-B introduite initialement.

Comme indiqué plus haut, l'acide triflique, grâce à sa grande réactivité, induit un temps de séjour plus faible dans la zone de réaction. Ainsi, lorsque le procédé est effectué en mode batch, la quantité de sous-produits est réduite et le rendement du procédé amélioré. La mise en oeuvre du procédé de l'invention en mode « batch » (par lots) et en utilisant l'acide triflique est donc particulièrement intéressante.

Par ailleurs, lorsque le procédé est effectué en continu, l'acide triflique, de par son faible temps de séjour dans la zone de réaction, induit un encombrement plus réduit, ce qui est avantageux en termes d'équipement.

L'utilisation de l'acide triflique permet donc d'effectuer le procédé en mode batch ou en mode continu, dans des réacteurs séparés (réaction de transanhydrification puis extraction) ou dans une seule colonne de distillation avec des flux croisés de réactifs et des effluents.

Selon un mode de réalisation, un ou plusieurs inhibiteurs de polymérisation peuvent être introduits au cours du procédé afin de limiter la formation de sous-produits tels que des polymères à base d'acide (méth)acrylique et/ou d'anhydride (méth)acrylique.

Ils peuvent alors être introduits avec l'acide A-COOH dans la zone de réaction et/ou dans la zone d'extraction.

Avantageusement, ces inhibiteurs sont introduits dans la zone d'extraction. En effet, ils sont particulièrement efficaces dans la zone d'extraction et permettent notamment d'éviter les réactions de condensation susceptibles de s'y produire.

De préférence, lorsque la zone d'extraction consiste en une colonne de distillation, les inhibiteurs sont introduits en tête de colonne.

Les inhibiteurs doivent être actifs vis-à-vis de la polymérisation tout en étant inertes à l'égard des anhydrides et de l'acide (méth)acrylique.

Ils peuvent notamment être choisis parmi l'hydroquinone, l'hydroquinone monométhyl éther, le topanol A, la phénothiazine et l'hydroxytétraméthylpipéridinoxyl (hydroxy-TEMPO).

L'invention sera mieux comprise à la lecture de la description qui va suivre, donnée uniquement à titre d'exemple, et faite en se référant aux dessins annexés, sur lesquels :

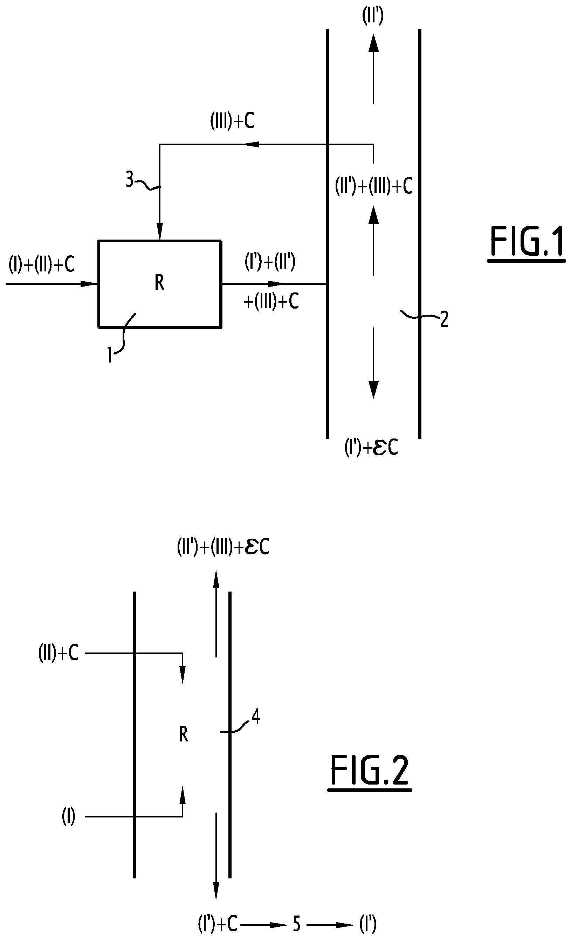

- la Figure 1 est une vue en coupe suivant un plan vertical médian d'un dispositif dans lequel le procédé selon l'invention est mis en oeuvre, constitué d'un réacteur 1 relié à une colonne de distillation 2, de nouveau reliée au réacteur 1 par une boucle de réinjection 3. Le réacteur 1 comprend une zone de réaction R.

L'anhydride B-C(=O)-O-(O=)C-B, noté (I), l'acide A-COOH, noté (II), et un catalyseur C, plus volatil que l'anhydride A-C(=O)-O-(O=)C-A, noté (I'), sont injectés dans le réacteur 1. L'étape a) se déroule dans la zone de réaction R et conduit à la formation de l'anhydride mixte A-C(=O)-O-(O=)C-B, noté (III), et l'acide B-COOH, noté (II'). Afin que l'étape b) puisse avoir lieu, l'acide (II') est éliminé au fur et à mesure pour que sa quantité dans la zone R soit inférieure à celle de l'acide (II). L'étape b) se déroule alors dans la zone R et conduit à la formation de l'anhydride (I').

A la sortie du réacteur 1, les réactifs (I), (II) et C, le composé intermédiaire (III) et les produits (I') et (II') sont acheminés vers la colonne de distillation 2. L'anhydride (I') étant moins volatil que tous les autres composés, l'acide (II'), l'anhydride mixte (III), ainsi que le catalyseur C sont aisément extraits du mélange. L'acide (II') est récupéré en tête de colonne, et l'anhydride (III) et la majorité du catalyseur C sont récupérés et réinjectés par l'intermédiaire de la boucle 3, dans le réacteur 1. Les réactifs (I) et (II) sont également récupérés et réinjectés via la boucle 3 dans le réacteur 1. L'anhydride (I') est récupéré en pied de colonne avec une très faible quantité de catalyseur C (εC), typiquement inférieure à 10 ppm, voire 5 ppm, et donc non réactive.
- la Figure 2 est une vue en coupe suivant un plan vertical médian d'un dispositif constitué d'une unique colonne de distillation 4, dans laquelle le procédé selon l'invention est mis en oeuvre, sous forme de distillation réactive.

L'acide A-COOH, noté (II) et le catalyseur C sont injectés en haut de la colonne 4, et l'anhydride B-C(=O)-O-(O=)C-B, noté (I) est injecté en bas de la colonne 4. Les divers réactifs se rencontrent dans la zone de réaction R.

L'étape a) se déroule et conduit à la formation de l'anhydride mixte A-C(=O)-O-(O=)C-B, noté (III), et l'acide B-COOH, noté (II'). Du fait des différences de volatilité qui existent, l'acide (II') est systématiquement éliminé au fur et à mesure et sa quantité dans la zone R est donc inférieure à celle de l'acide (II). L'étape b) peut alors se dérouler et conduit à la formation de l'anhydride A-C(=O)-O-(O=)C-A, noté (I').

Au fur et à mesure que se déroulent les étapes a) et b), l'acide (II') et l'anhydride mixte (III), plus volatils que l'anhydride (I'), sont extraits et récupérés en tête de colonne. La majorité du catalyseur C, bien qu'il soit plus volatil que l'anhydride (I'), est entraînée vers le pied de colonne, mélangé à l'anhydride (I'). Ainsi, seule une faible quantité de catalyseur C est récupérée en tête de colonne (εC). Après avoir été récupérés en pied de colonne, l'anhydride (I') et le catalyseur C peuvent être séparés dans un dispositif 5, par exemple une deuxième colonne de distillation, afin d'isoler l'anhydride (I').

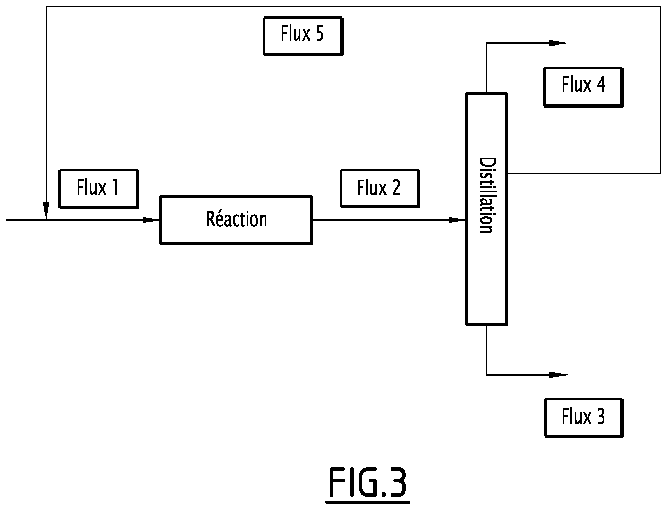

- la Figure 3 est un schéma du dispositif utilisé dans l'Exemple 3.

### EXEMPLES

### Exemple 1 :

Dans un réacteur en verre de volume 1 litre agité mécaniquement et à double enveloppe, on a introduit 200 g d'anhydride acétique, 337 g d'acide méthacrylique, 2,68 g de phénothiazine et une quantité connue d'acide triflique, indiquée dans le Tableau 1. La température a été maintenue entre 85°C et 95°C en fonction des essais, à la pression atmosphérique.

Des prélèvements au cours du temps ont ensuite été effectués afin de comparer les essais entre eux en ce qui concerne le temps pour atteindre l'équilibre.

| N°de l'essai | Quantité d'acide triflique | Ratio molaire AM/Ac2O | Température (°C) | Temps pour atteindre l'équilibre (min) |
|---|---|---|---|---|
| 1 | 0 | 2 | 85 | 105 |
| 2 | 25 ppm | 2 | 85 | 17 |
| 3 | 50 ppm | 2 | 85 | 12 |
| 4 | 50 ppm | 2 | 95 | 7 |

### Exemple 2 :

L'alimentation des réactifs et les conditions opératoires sont celles décrites dans l'essai 4 de l'Exemple 1. On a également ajouté de la phénothiazine comme inhibiteur de polymérisation.

En revanche, la réaction a été conduite en continu dans un réacteur tubulaire de 1/8 de diamètre et une longueur suffisante pour atteindre l'équilibre à la sortie. Ce flux a servi à alimenter une colonne de 10 à 15 plateaux. La colonne faisait 3 cm de diamètre et le vide de 20 mbar a été assuré par une pompe à palette.

Les 537 g/h de produit brut qui ont été obtenus se partagent comme suit : 116 g/h en pied de colonne et 421 g/h dans le reste de la colonne. Le produit récupéré en pied contenait de l'anhydride méthacrylique à 96%, de l'anhydride mixte (A-C(=O)-O-(O=)C-B) à 1,5%, 1% d'inhibiteur et 1,5% de composés inconnus.

Une alimentation en continu de phénothiazine a été mise en oeuvre en tête de colonne pour éviter une polymérisation dans la colonne. La pression de travail était de 20 mbar en tête et la température du pied était de 93°C.

### Exemple 3 :

De l'anhydride acrylique CH₂=CH-C(=O)-O-(O=)C-CH=CH a été préparé selon le procédé de l'invention, par réaction entre l'acide acrylique et l'anhydride acétique. L'enchaînement des équipements est représenté sur la Figure 3.

Dans la zone de réaction, la température de réaction a été fixée à 100°C et la pression à la pression atmosphérique.

Dans la zone de distillation (extraction), la pression a été fixée à 20 mbar absolu et un gradient de température (entre 90°C en pied de colonne et 25°C en tête de colonne) a été observé.

Comme indiqué sur la Figure 3, la zone de réaction a été alimentée par le flux 1, d'un débit global de 2 kg/h, et composé du flux de recyclage 5 et d'une alimentation continue d'un mélange frais d'anhydride acétique, acide acrylique et acide triflique.

Les ajustements du flux 1, composé du flux de mélange frais et du flux 5, ont été tels que dans la zone de réaction, il y avait en permanence :
- de l'anhydride acétique : débit d'entrée de 795 g/h (7,794 mol/h),
- de l'acide acrylique : débit d'entrée de 1004 g/h (13,95 mol/h), et
- de l'acide triflique à 50 ppm en poids par rapport à l'ensemble {anhydride acétique + acide acrylique}.

En sortie de cette zone de réaction, le flux 2 acheminait essentiellement de l'anhydride acrylique, de l'acide acétique, de l'acide triflique et de l'anhydride mixte acrylique/acétique, vers la colonne de distillation.

Dans les conditions décrites ci-dessus, l'anhydride acrylique, d'une pureté supérieure à 95%, a été récupéré dans le flux 3 avec un débit d'environ 840 g/h (6,66 mol/h). En tête de colonne, le flux 4 (950 g/h, 14,8 mol/h) était composé très majoritairement (> 95% poids) d'acide acétique. Le flux de recyclage 5 transportait essentiellement de l'acide triflique et de l'anhydride mixte acrylique/acétique pour les réinjecter dans la zone de réaction.

## Revendications

1. Procédé de préparation d'un anhydride de formule A-C(=O)-O-(O=)C-A, où A est -CR=CH₂ et R est -H ou -CH₃, comprenant :
a) une étape de réaction d'un anhydride B-C(=O)-O-(O=)C-B avec un acide A-COOH, A étant tel que défini ci-dessus, cette étape conduisant à la formation d'un anhydride A-C(=O)-O-(O=)C-B et d'un acide B-COOH, A et B étant tels que ledit acide B-COOH est plus volatil que ledit acide A-COOH, et
b) une étape de réaction dudit anhydride A-C(=O)-O-(O=)C-B avec l'acide A-COOH dans des conditions telles que la quantité d'acide B-COOH est inférieure à la quantité d'acide A-COOH, conduisant à la formation de l'anhydride A-C(=O)-O-(O= )C-A,
dans lequel lesdites étapes de réaction sont mises en oeuvre en présence d'un catalyseur acide plus volatil que ledit anhydride A-C(=O)-O-(O=)C-A,
choisi parmi l'acide trifluoroacétique, le triflimide et l'acide triflique.

2. Procédé selon la revendication 1, dans lequel l'acide B-COOH est éliminé au cours des étapes de réaction a) et b).

3. Procédé selon la revendication 1 ou 2, effectué en continu.

4. Procédé selon l'une quelconque des revendications 1 à 3, dans lequel la masse molaire de B est inférieure à la masse molaire de A.

5. Procédé selon l'une quelconque des revendications 1 à 4, dans lequel B est un groupe méthyle ou éthyle, et notamment un groupe méthyle.

6. Procédé selon l'une quelconque des revendications 1 à 5, dans lequel le catalyseur acide est l'acide triflique.

7. Procédé selon l'une quelconque des revendications 1 à 6, dans lequel les étapes de réaction a) et b) s'effectuent à une température de 60°C à 120°C, avantageusement de 70°C à 110°C, de préférence de 90°C à 100°C.

8. Procédé selon l'une quelconque des revendications 1 à 7, dans lequel les étapes de réaction a) et b) s'effectuent à une pression de 0,01 bar à 3 bar, avantageusement de 0,5 bar à 1,5 bar, de préférence à pression atmosphérique.

9. Procédé selon l'une quelconque des revendications 1 à 8, comprenant une étape d'extraction du catalyseur acide et de l'acide B-COOH formé, pour séparer le catalyseur acide et l'acide B-COOH, notamment par distillation.

10. Procédé selon la revendication 9, dans lequel les étapes de réaction sont effectuées dans un réacteur et l'étape d'extraction est effectuée dans une ou plusieurs colonnes de distillation successives, distinctes du réacteur.

11. Procédé selon la revendication 9, dans lequel les étapes de réaction et l'étape d'extraction sont effectuées dans une ou plusieurs colonnes de distillation successives.

## Patentansprüche

1. Verfahren zur Herstellung eines Anhydrids der Formel A-C(=0)-0-(0=)C-A, wobei A für -CR=CH₂ steht und R für -H oder -CH₃ steht, umfassend:
a) einen Schritt der Umsetzung eines Anhydrids B-C(=0)-0-(0=)C-B mit einer Säure A-COOH, wobei A wie oben definiert ist, wobei dieser Schritt zur Bildung eines Anhydrids A-C(=0)-0-(0=)C-B und einer Säure B-COOH führt, wobei A und B so beschaffen sind, dass die Säure B-COOH flüchtiger ist als die Säure A-COOH, und
b) einen Schritt der Umsetzung des Anhydrids A-C(=O)-O-(O=)C-B mit der Säure A-COOH unter solchen Bedingungen, dass die Menge der Säure B-COOH kleiner ist als die Menge der Säure A-COOH, was zur Bildung des Anhydrids A-C(=0)-0-(0=)C-A führt, wobei die Umsetzungsschritte in Gegenwart eines sauren Katalysators, der flüchtiger ist als das Anhydrid A-C(=O)-O-(O=)C-A und aus Trifluoressigsäure, Triflimid und Trifluormethansulfonsäure ausgewählt wird, durchgeführt werden.

2. Verfahren nach Anspruch 1, bei dem die Säure B-COOH im Lauf der Umsetzungsschritte a) und b) entfernt wird.

3. Verfahren nach Anspruch 1 oder 2, das kontinuierlich durchgeführt wird.

4. Verfahren nach einem der Ansprüche 1 bis 3, bei dem die Molmasse von B kleiner ist als die Molmasse von A.

5. Verfahren nach einem der Ansprüche 1 bis 4, bei dem es sich bei B ist eine Methyl- oder Ethylgruppe und insbesondere eine Methylgruppe.

6. Verfahren nach einem der Ansprüche 1 bis 5, bei dem es sich bei dem sauren Katalysator um Trifluormethansulfonsäure handelt.

7. Verfahren nach einem der Ansprüche 1 bis 6, bei dem die Umsetzungsschritte a) und b) bei einer Temperatur von 60°C bis 120°C, vorteilhafterweise von 70°C bis 110°C, vorzugsweise von 90°C bis 100°C, durchgeführt werden.

8. Verfahren nach einem der Ansprüche 1 bis 7, bei dem die Umsetzungsschritte a) und b) bei einem Druck von 0,01 bar bis 3 bar, vorteilhafterweise von 0,5 bar bis 1,5 bar, vorzugsweise bei Normaldruck, durchgeführt werden.

9. Verfahren nach einem der Ansprüche 1 bis 8, umfassend einen Schritt des Entfernens des sauren Katalysators und der gebildeten Säure B-COOH zur Abtrennung des sauren Katalysators und der Säure B-COOH, insbesondere durch Destillation.

10. Verfahren nach Anspruch 9, bei dem die Umsetzungsschritte in einem Reaktor durchgeführt werden und der Entfernungsschritt in einer oder mehreren aufeinanderfolgenden Destillationssäulen, die von dem Reaktor verschieden sind, durchgeführt wird.

11. Verfahren nach Anspruch 9, bei dem die Umsetzungsschritte und der Entfernungsschritt in einer oder mehreren aufeinanderfolgenden Destillationssäulen durchgeführt werden.

## Claims

1. Process for the preparation of an anhydride of formula A-C(=O)-O-(O=)C-A, where A is -CR=CH₂ and R is -H or -CH₃, comprising:
a) a stage of reaction of an anhydride B-C(=O)-O-(O=)C-B with an acid A-COOH, A being as defined above, this stage resulting in the formation of an anhydride A-C(=O)-O-(O=)C-B and of an acid B-COOH, A and B being such that said acid B-COOH is more volatile than said acid A-COOH, and
b) a stage of reaction of said anhydride A-C(=0)-0-(0=)C-B with the acid A-COOH under conditions such that the amount of acid B-COOH is less than the amount of acid A-COOH, resulting in the formation of the anhydride A-C(=0)-0-(0=)C-A, in which said reaction stages are carried out in the presence of an acid catalyst which is more volatile than said anhydride A-C(=0)-0-(0=)C-A and is chosen from trifluoroacetic acid, triflimide and triflic acid.

2. Process according to Claim 1, in which the acid B-COOH is removed during the reaction stages a) and b).

3. Process according to Claim 1 or 2, carried out continuously.

4. Process according to any one of Claims 1 to 3, in which the molar mass of B is less than the molar mass of A.

5. Process according to any one of Claims 1 to 4, in which B is a methyl or ethyl group and in particular a methyl group.

6. Process according to any one of Claims 1 to 5, in which the acid catalyst is triflic acid.

7. Process according to any one of Claims 1 to 6, in which the reaction stages a) and b) are carried out at a temperature of 60°C to 120°C, advantageously of 70°C to 110°C and preferably of 90°C to 100°C.

8. Process according to any one of Claims 1 to 7, in which the reaction stages a) and b) are carried out at a pressure of 0.01 bar to 3 bar, advantageously of 0.5 bar to 1.5 bar and preferably at atmospheric pressure.

9. Process according to any one of Claims 1 to 8, comprising a stage of extraction of the acid catalyst and of the acid B-COOH formed, in order to separate the acid catalyst and the acid B-COOH, in particular by distillation.

10. Process according to Claim 9, in which the reaction stages are carried out in a reactor and the extraction stage is carried out in one or more successive distillation columns separate from the reactor.

11. Process according to Claim 9, in which the reaction stages and the extraction stage are carried out in one or more successive distillation columns.
